(19) Europäisches Patentamt
European Patent Office
Office européen des brevets

(11) Publication number: 0 228 666 A2

# (12) EUROPEAN PATENT APPLICATION

(21) Application number: 86117642.8

(22) Date of filing: 18.12.86

(51) Int. Cl.4: G01N 33/579 , C07K 5/04 , C07K 7/04 , C07K 15/00

(30) Priority: 06.01.86 FI 860043

(43) Date of publication of application: 15.07.87 Bulletin 87/29

(84) Designated Contracting States: AT BE CH DE ES FR GB GR IT LI NL SE

(71) Applicant: Orion Corporation Ltd
Saunatontuntie 1
SF-02100 Espoo(FI)

(72) Inventor: Lundström, Erkki Matias
Paatsamakuja 6 C 34
FI-01360 Vantaa(FI)
Inventor: Rantama, Annikka Helena
Lystimäenkuja 5,
FI-02210 Espoo(FI)
Inventor: Raussi, Jaana Heleni
Soittajantie 3 D 25,
FI-00420 Helsinki(FI)

(74) Representative: Vossius & Partner
Siebertstrasse 4 P.O. Box 86 07 67
D-8000 München 86(DE)

## (54) Peptide substrates and method for the quantitative assay of endotoxin.

(57) The subject matter of the invention is a peptide-based compound for utilization in the assay of endotoxin in samples by an LAL-type analysis method. The structure of the compound has the form:

X -A1 -A2 -A3 -B -R

in which X is hydrogen or a protective aromatic hydrocarbon or acyl group; A1 is a peptide chain consisting of 0-100 amino acids; A2 is glycine; A3 is arginine; B is an amide mixture and R is a molecule which, after its liberation in the LAL-type analysis, can be identified by diazotizing its liberated amine group and allowing it to react with a compound with which it combines to form a visibly coloured final product.

EP 0 228 666 A2

# PEPTIDE SUBSTRATES AND METHOD FOR THE QUANTITATIVE ASSAY OF ENDOTOXIN

The invention consists of peptide substrates and their utilization in endotoxin assays in which a lipopolysaccharide activates the coagulation factor enzyme found in the blood cell lysates of many arthropods (Crustacea) and insects (Insecta).

It has been known for many years that bacterial endotoxins activate the blood clotting enzyme occurring in amebocyte lysates of horseshoe crabs. This property has been described in many publications, eg. J. Levin and F. B. Bangs, 1968: "Clottable Protein in Limulus: Its localization and kinetics of its coagulation by endotoxin", Thomb. Diath. Haemorrh. 19, 186 and S. Nakamura, 1976: "Amino acid sequence studies on the fragments produced from horseshoe crab coagulogen during gel formation: Homologies with primate fibrino-peptide B", Biochemical and Biophysical Research Communication, 72, 902. These investigations demonstrated that the coagulation of Limulus amebocyte lysate (LAL) requires activation of the proclotting enzyme by endotoxin in the presence of divalent cations. The active enzyme thus formed cleaves coagulogen at the C-carboxyl terminal between the glycine and arginine subunits. The cleaved coaculogen molecules polymerize and thus bring about coagulation. The first endotoxin tests were based on observation of the gel thus formed.

The LAL name has been used for the amebocyte lysates of both American horseshoe crabs (Limulus polyphemus) and the Japanese species Tachypleus tridentatus.

LAL endotoxin tests are extremely sensitive. Tests based on coagulation may be used to detect amounts of endotoxin as low as $10^{-12}$ g/ml.

Fluorogenic or chromogenic substrates have been used for the assay of many enzymes, eg. trypsin, thrombin, thromboblastin, serine protease, plasmin and plasminogen. It was also realized that fluorogens or chromogens can be used as LAL-substrates. This subject has been discussed both in the scientific literature, eg. Iwanaga et al., 1978: "Chromogenic substrate for horseshoe crab clotting enzyme: Its application for the assay of bacterial endotoxin", Homeostatis, 7, 183 and in the patent publications US 4188265, US 4406832 and US 4510241. In these methods the peptide substrates are composed of a protecting group, a peptide chain and the chromogen or fluorogen. The structure of the substrate has the form:

R1 -A1 -A2 -A3 -A4 -X

in which R1 is the protecting group, A1 is isoleucine, valine or leucine or else is absent, A2 is glutamic acid or asparaginic acid or else is absent, A3 is alanine, cysteine or glycine, A4 is arginine and X is the fluorogen or chromogen. The protecting group may be eg. a benzoyl-, carbobenzoxy-, tertiary butoxycarbonyl-, p-toluenesulphonyl-, acetyl-or an alcanoyl group containing 1-12 carbon atoms. The benzoyl group may be substituted by eg. one of the following groups: halogens or short-chain alkyl-, amino-or phenyl groups. The chromogens or fluorogens used have included nitrophenyl-, methyl nitrophenyl-, dinitrophenyl-, naphthylnitronaphthyl-, metoxynaphthyl-, indoxyl-, methyl indoxyl-, 4-methyl-umbelliferyl-or resorphine groups.

In this invention the chromogen or fluorogen is replaced by a compound which is colourless both when attached to a peptide chain and when occurring in the free state. The compound is attached to the peptide chain and is released by enzymic activity. The released compound has the form:

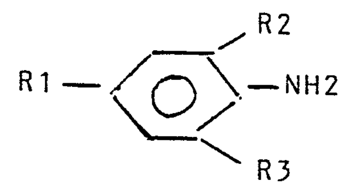

in which R1, R2 and R3 are one of the following substituents: H, halogen, secondary or tertiary amine,

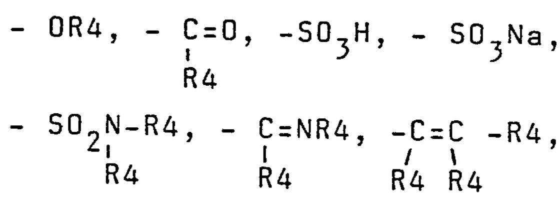

in which R4 is an aryl-or alkyl group or hydrogen. After the enzymatic reaction the released compound is diazotized, as a result of which a diazo salt is formed, eg:

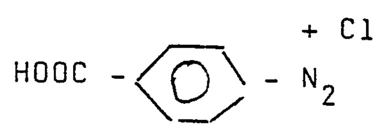

After diazotization the diazo salt is allowed to react with a coupling reagent, with the production of visible colour. Such coupling reagents include eg. the disodium salt of $\beta$-amino-1-naphthalene-3,6-disulphonic acid or N-(1-naphthyl) ethylene diamine dihydrochloride. The coupling reagent may be absorbed onto eg. absorbent paper, which may be attached to the end of a plastic stick. Thus the test result can be read from the colour formed at the end of the test stick.

In traditional LAL-tests there has been used as the substrate, peptide chains into which a chromogenic group, usually paranitroaniline, has been included. Paranitroaniline is yellow in colour, thus making it rather difficult to assess in urine samples.

In the test described in this invention the visible colour produced is either red or blue. The test can be used to detect smaller concentrations than those detectable using earlier methods, because the formation of the diazo colouring is extremely sensitive and because the colour formed is more easily distinguishable against the background colouring of eg. urine than is that of paranitroaniline.

Example 1

25 µl of LAL-reagent (commercial reagent, eg. Pyrotest, Difco Rabo, USA) is pipetted to a test tube. To the LAL-reagent is pipetted 25 µl of sample (urine). The mixture is stirred and incubated for 10 min at 37°C. 50 µl of substrate (prepared by a normal peptide synthesis method) is added and the mixture is incubated at 37°C for a further 3 min. After this, 20 µl 0.5M trichloracetic acid (TCA) and 20 µl of an aqueous solution of 0.05 mg/ml sodium nitrite are added. Finally, 20 µl of a 10 mg/ml solution of 8-amino-1-naphthol-3,6-disulphonic acid (disodium salt, Fluka) in 0.5 M sodium bicarbonate is added. The result is assessed immediately: A red colouration indicates a positive sample.

Example 2

25 µl of LAL-reagent (commercial reagent, eg. Pyrotest, Difco Rabo, USA) is pipetted to a test tube. To the LAL-reagent is pipetted 25 µl of sample (urine). The mixture is stirred and incubated for 10 min at 37°C. 50 µl of substrate - (prepared by a normal peptide synthesis method) is added and the mixture is incubated at 37°C for a further 3 min. After this, 20 µl 0.05 M TCA and 20 µl of an aqueous solution of 0.05 mg/ml sodium nitrite are added. Finally, 20 µl of a 0.75 % aqueous solution of N-(1-naphthyl) ethylene diamine dihydrochloride is added. The result is assessed immediately: a red colouration indicates a positive sample.

## Claims

1. A peptide-based compound for utilization in the assay of endotoxin in samples by an LAL-type analysis method, wherein the structure of the compound has the form:

X -A1 -A2 -A3 -B -R

in which X is hydrogen or a protective aromatic hydrocarbon or acyl group; A1 is a peptide chain consisting of 0-100 amino acids; A2 is glycine; A3 is arginine; B is an amide mixture and R is a molecule which, after its liberation in the LAL-type analysis, can be identified by diazotizing its liberated amine group and allowing it to react with a compound with which it combines to form a visibly coloured final product.

2. A peptide compound according to claim 1 in which R is:

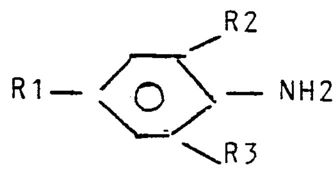

in which R1, R2 and R3 are one of the following substituents: H, halogen, secondary or tertiary amine, -OR4,- 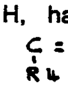, $-SO_3H$, $-SO_3Na$,

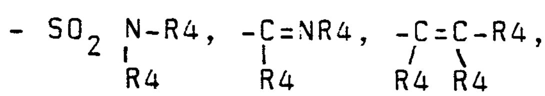

in which R4 is an aryl-or alkyl group or hydrogen.

3. A method for carrying out LAL-type analysis, wherein the assay substrate is a peptide-based compound of the structure:

X -A1 -A2 -A3 -B -R

in which X is hydrogen or a protective aromatic hydrocarbon or acyl group; A1 is a peptide chain consisting of 0-100 amino acids; A2 is glycine; A3 is arginine; B is an amide mixture and R is a molecule which, after its liberation in the LAL-type analysis, can be identified by diazotizing the liberated amine group and allowing it to react with a compound with which it combines to form a visibly coloured final product.

4. A method according to claim 3 in which R is:

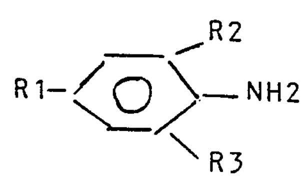

in which R1, R2 and R3 are one of the following substituents: H, halogen, secondary or tertiary amine, -OR4,- 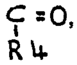 $SO_3H$, $-SO_3Na$,

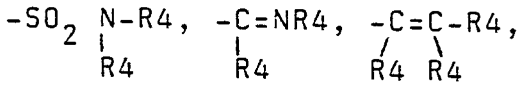

in which R4 is an aryl -or alkyl group or hydrogen.

5. A method according to claims 3-4, wherein the diazotized compound is allowed to react with 8-amino-1-naphthol-3,6-disulphonic acid (disodium salt).